# EUROPÄISCHE PATENTSCHRIFT

(11) **EP 3 319 834 B1**
(45) Veröffentlichungstag und Bekanntmachung des Hinweises auf die Patenterteilung: **18.08.2021**
(21) Anmeldenummer: 16711301.8
(22) Anmeldetag: 23.03.2016
(51) Int. Cl.: B60N 2/90, A61H 23/02

(54) **VERFAHREN ZUR ANSTEUERUNG VON MASSAGEEINHEITEN IN EINEM SITZ, STEUERVORRICHTUNG ZUR DURCHFÜHRUNG UND SITZANORDNUNG**
METHOD FOR DRIVING MASSAGE UNITS IN A SEAT, CONTROLLING DEVICE FOR CARRYING OUT AND SEAT ARRANGEMENT
PROCÉDÉ DE PILOTAGE POUR UNITÉ DE MASSAGE DANS UN SIÈGE, DISPOSITIF DE COMMANDE POUR EFFECTUER ET AGENCEMENT DE SIÈGE

(30) Priorität: 07.08.2015 DE 102015215171
(43) Veröffentlichungstag der Anmeldung: 16.05.2018
(73) Patentinhaber: Brose Fahrzeugteile SE & Co. Kommanditgesellschaft, Coburg, 96450 Coburg (DE)
(72) Erfinder: MERGL, Christian, 97475 Zeil/Main (DE); FALINSKI, Wojciech, 96450 Coburg (DE); KARL, Christian, 96450 Coburg (DE); JUERGENLIEMK, Martin, 96253 Untersiemau (DE); UEBEL, Wolfgang, 96479 Weitramsdorf (DE); HERRMANN, Christian, 96269 Großheirath (DE); POHL, Florian, 96237 Ebersdorf (DE)
(74) Vertreter: 2K Patentanwälte Blasberg Kewitz & Reichel
(86) Internationale Anmeldenummer: PCT/EP2016/056388
(87) Internationale Veröffentlichungsnummer: WO 2017/025204

(56) Entgegenhaltungen:
- EP-A1- 1 350 502
- DE-A1-102009 022 955
- DE-A1-102013 216 885
- JP-A- 2010 011 923
- KR-A- 20120 136 173
- US-A- 5 951 500
- US-A- 6 027 463

## Beschreibung

### GEBIET DER ERFINDUNG

Die vorliegende Erfindung betrifft ein Fahrzeug-Audio-System und Verfahren zur Ansteuerung von Massageeinheiten in einem Sitz.

### TECHNISCHER HINTERGRUND

Zur Ansteuerung von Massageeinheiten in einem Sitz existieren Verfahren, die ein Ein- und Ausschalten oder das Abspielen eines fest vorgegebenen Programmablaufs vorsehen.

Die DE 297 05530 U1 beschreibt einen Fahrzeugsitz, bei dem Schwingköpfe integriert sind, deren Antriebsfrequenz durch entsprechende Benutzereingabe in unterschiedlichen Stufen oder stufenlos einstellbar ist. Eine derartige Steuerung stellt jedoch lediglich monotone Massageabläufe bereit.

Die DE 43 31 663 C1 beschreibt eine die positionsverstellenden und die konturverstellenden Mittel eines Fahrzeugsitzes gemeinsam ansteuernde Steuerungseinrichtung. Diese weist eine Speichereinheit auf, in der Ansteuerprogramme für diese Mittel speicherbar sind. Die Konturverstellmittel werden gleichzeitig gemeinsam von der Steuerungseinrichtung in einer vorbestimmbaren zeitlichen Abfolge zur Bewirkung eines Massage- und Gymnastikprogramms aktiviert. Nachteilig sind derartige Programme einem Benutzer relativ schnell bekannt.

Die EP1350502 beschreibt einen Massageapparat, der den Rhythmus oder die Melodie einer Musikquelle einbezieht.

Die US6027463 beschreibt ein Massagesystem, das mit einem Spektrum von Audiosignalen interagiert

Die US5951500 beschreibt ein weiteres Audio-responsives Massagesystem.

Die DE 10 2009 022 955 beschreibt eine Sitzmassage-Einrichtung an einem Fahrzeugsitz.

Die DE 10 2013 216 885 A1 beschreibt einen Fahrzeugsitz mit einer Massageeinrichtung und mindestens einer Heizeinrichtung zur Umsetzung einer "Hot-Stone"-Massagefunktion".

Die JP 2010 011923 A beschreibt ein Massagegerät und ein mit diesem Massagegerät ausgerüstetes Fahrzeug.

Die KR 2012 0136173 A beschreibt einen akustischen Aktuator für einen Sitz.

### ZUSAMMENFASSUNG DER ERFINDUNG

Vor diesem Hintergrund liegt der vorliegenden Erfindung die Aufgabe zugrunde, ein verbessertes Verfahren zur Ansteuerung von Massageeinheiten in einem Sitz anzugeben.

Erfindungsgemäß wird diese Aufgabe durch ein Fahrzeug-Audio-System mit den Merkmalen des Patentanspruchs 1 und/oder durch ein Verfahren mit den Merkmalen des Anspruchs 2 gelöst.

Die der vorliegenden Erfindung zugrunde liegende Idee besteht darin, ein einem Insassen zur Unterhaltung bereitgestelltes primäres Audiosignal (akustisches Unterhaltungssignal), insbesondere eines Musikstücks, als Eingangsgröße einer Steuervorrichtung zum Steuern von in einem Sitz integrierten Massageeinheiten zu verwenden.

Auf diese Weise ist es erfindungsgemäß ermöglicht, ein völlig neuartiges durch einen Fahrzeugsitz erbringbares Massageerlebnis zu erzeugen, bei welchem gleichzeitig aufeinander abgestimmte akustische und taktile Reize in beliebiger Vielfalt gesetzt werden können. Ein Massagesystem ist somit erfindungsgemäß vorteilhaft für weitere Applikationen im Fahrzeug nutzbar.

Sofern dabei Musik als primäres Unterhaltungssignal dient, wird das Hörerlebnis der Musik durch die vom Sitz übertragene Massage erweitert. Die Musik kann erfindungsgemäß über die in den Sitz integrierten Massageeinheiten von einem Insassen in Form einer Rücken- und/oder Gesäß- und/oder Nacken und/oder Kopfmassage, insbesondere an einem Großteil des Körpers, zusätzlich taktil gespürt werden. Somit wird die Musik insgesamt intensiver empfunden.

Ferner wird, sofern die Musik gleichzeitig über Lautsprecher ausgegeben wird, durch die mit der Musik gesetzten akustischen Reize auch die mit den Massageeinheiten erzeugte Massage intensiver empfunden.

Da die akustischen und taktilen Reize aufeinander abgestimmt sind entsteht erfindungsgemäß somit insgesamt ein intensiveres Erlebnis, als wenn Massage und Musik unabhängig voneinander ablaufen.

Ferner wird das Programm nicht bekannt oder "langweilig", da der Programmablauf durch Einstellen einer anderen Musik beliebig variierbar ist.

Insbesondere findet somit erfindungsgemäß eine Kopplung des des Unterhaltungssystems eines Fahrzeugs, ggfs. ausgebildet als Infotainmentsystem, mit der Massagefunktion eines Fahrzeugsitzes statt.

Zum Abnehmen des Unterhaltungssignals von der Abspielvorrichtung kann ein Abnehmer vorgesehen sein. Ebenso ist eine digitale Datenübertragung des Unterhaltungssignals von der Abspielvorrichtung zur Steuervorrichtung möglich. Das abgenommene oder übertragene Unterhaltungssignal wird dann als Eingangsgröße für die Steuervorrichtung verwendet.

Eine Anzahl von Massageeinheiten kann eine Einzahl oder eine Vielzahl darstellen.

Das erfindungsgemäße Verfahren kann zusätzlich zu bekannten Ansteuerungen von Massageeinheiten, insbesondere als Zusatzfunktion, vorgesehen sein.

Vorteilhafte Ausgestaltungen und Weiterbildungen ergeben sich aus den weiteren Unteransprüchen sowie aus der Beschreibung unter Bezugnahme auf die Figuren der Zeichnung.

Gemäß einer Ausführungsform wird das akustische Unterhaltungssignal in vorbestimmte Frequenzbereiche aufgeschlüsselt. Zumindest ein vorbestimmter Frequenzbereich des akustischen Unterhaltungssignals wird dabei zu einem Ausgangssignal für zumindest eine Massageeinheit gewandelt. Dementsprechend können erfindungsgemäß vorbestimmte Frequenzbereiche oder auch nur ein vorbestimmter Frequenzbereich zum Steuern von in einem Sitz integrierten Massageeinheiten genutzt werden. Der vorbestimmte Frequenzbereich kann oder die vorbestimmten Frequenzbereiche können an die Art bzw. das Genre der Musik anpassbar sein. Vorteilhaft kann somit der am besten zu der Musik passende und/oder der am besten in eine Massage übersetzbare Frequenzbereich genutzt werden.

Ferner kann gemäß einer Weiterbildung eine an die Art des Unterhaltungssignals, im Falle von Musik an beispielsweise and die Musikart bzw. an das Genre, angepasste Verstärkung und Auswahl der verschiedenen Frequenzbereiche vorgesehen sein. Auf diese Weise kann somit beispielsweise bei Techno oder Klassik als Unterhaltungssignal eine daran angepasste Ansteuerung der Massageeinheiten erfolgen und so das Hörerlebnis der jeweiligen Musik durch die vom Sitz übertragene Massage an die Musikart angepasst erweitert werden.

Gemäß einer Ausführungsform werden verschiedene Frequenzbereiche des akustischen Unterhaltungssignals jeweils zu einem Ausgangssignal gewandelt und zur Ansteuerung verschiedener Massageeinheiten verwendet. Somit wird vorteilhaft die Musik in einer aufgeschlüsselten Form spürbar. Die Massage wird dadurch vielfältiger und interessanter.

Gemäß einer Ausführungsform ist eine Vielzahl von Massageeinheiten über die Sitzfläche des Sitzes verteilt angeordnet. Die den verschiedenen Frequenzbereichen des akustischen Unterhaltungssignals zugeordneten Ausgangssignale werden dabei zur Ansteuerung von an unterschiedlichen Stellen angeordneten Massageeinheiten ausgegeben. Es können dabei alle Frequenzen auf die unterschiedlichen Sitzbereiche verteilt in eine Massage, insbesondere eine Vibrationsmassage, gewandelt werden. Beispielsweise können tiefe Frequenzen, wie Bässe, beispielsweise mit Frequenzen von 80 bis 100 Hz, in einem unteren Bereich, insbesondere einem Sitzpolster oder Unterbau, und/oder höhere Frequenzen, wie Mitten und/oder Höhen, in einem oberen Bereich, insbesondere einer Rückenlehne, in eine Massage umgesetzt werden. Auch eine umgekehrte oder eine andere Verteilung ist möglich.

Gemäß einer Ausführungsform ist es denkbar, die Signale eines Mehrkanalsignals, beispielsweise eines Stereo- oder eines digitalen Mehrkanalsignals wie etwa ein 5.1-Signal, zur Ansteuerung von in einzelnen Bereichen des Sitzes zu nutzen. Insbesondere können auf diese Weise Massageeinheiten in einzelnen Seitenbereichen angesteuert werden. Somit kann das Mehrkanalsignal mittels einer Ansteuerung von an unterschiedlichen Stellen angeordneten Massageeinheiten durch die Massage abgebildet werden. Beispielsweise kann ein für einen linken Lautsprecher eines Stereo- oder 5.1-Systems bestimmtes Signal zu Ansteuerung von an der linken Seite des Sitzes angeordneten Massageeinheiten genutzt werden. Analog kann dies auch für einen rechten Bereich mit dem für einen rechten Lautsprecher vorgesehenen Signal erfolgen. Ferner sind Aufteilungen in mehr als zwei Bereiche, beispielsweise bei 5.1 Signalen in sechs Bereiche, denkbar.

Gemäß einer Ausführungsform wird lediglich ein Teil der in den Sitz integrierten Massageeinheiten mit dem auf dem Unterhaltungssignal basierenden Ausgangssignal angesteuert. Dementsprechend müssen nicht alle Massageeinheiten eines Sitzes oder eines Sitzbereichs mit durch das akustische Unterhaltungssignal gesteuerter Massage bzw. Vibration beaufschlagt werden. So kann beispielsweise ein Benutzer des Sitzes selbst die Bereiche, beispielsweise des Rückens bzw. der Beine, wählen, welche durch die Massageeinheiten massiert werden sollen. Die übrigen Massageeinheiten können stattdessen ruhen oder es kann stattdessen ein konventionelles Massageprogramm oder eine konstante Massage bzw. Vibration aktiv sein. Bei einer weiteren Ausführungsform werden alle Massageeinheiten eines Sitzes oder eines Sitzbereichs mit durch das akustische Unterhaltungssignal gesteuerter Massage bzw. Vibration beaufschlagt.

Gemäß einer Weiterbildung können aufeinander folgend stets wechselnde Motoren angesteuert werden. Vorzugsweise werden die Wechsel derart bzw. in einer Weise durchgeführt, dass sie für einen Insassen, das heißt eine auf dem Sitz sitzende Person, unspürbar sind. Somit wird stets nur ein Teil der Massageeinheiten gleichzeitig angesteuert. Eine Steuereinrichtung verteilt dazu Pegel zur Ansteuerung der Motoren der Massageeinheiten derart, dass nicht alle Motoren der in einem Sitz vorgesehenen Massageeinheiten gleichzeitig auf einen Pegel des Unterhaltungssignals reagieren. Stattdessen können sich die Motoren abwechseln und daher abwechselnd für eine vorbestimmte Zeitdauer, insbesondere zur Abkühlung der Motoren der Massageeinheiten, angehalten werden. Vorzugsweise werden die Motoren dabei derart angesteuert, dass die Abkühlphasen einzelner Motoren in ein auf dem Unterhaltungssignal basierendes Massageprogramm integrierbar sind, so dass die Abwechslung der Motoren für den Insassen unspürbar bzw. nicht wahrnehmbar ist. Somit wird trotz der Abwechslung eine gleichmäßige Massagewirkung erzielt.

Gemäß einer Ausführungsform wird das akustische Unterhaltungssignal unabhängig von einer an der Abspielvorrichtung eingestellten Ausgabelautstärke als Eingangsgröße für das Ansteuern verwendet. Dementsprechend kann das akustische Unterhaltungssignal gegebenenfalls auch gar nicht hörbar sein. Beispielsweise sind somit Betriebsmodi möglich, mit welchen im Falle von Musik als primärem Unterhaltungssignal Lieder anhand der Massage erraten werden können. Beispielsweise können von einem Insassen, insbesondere vom Fahrer oder von einem vorderen Beifahrer, verschiedene Lieder als Basis eines Massageprogramms gestartet werden. Die Lieder können dann von den anderen Insassen eines Fahrzeugs, beispielsweise von einer oder mehreren auf den Rücksitzen sitzenden Personen, erraten werden. Vorteilhaft ist somit ein zusätzliches Unterhaltungsangebot geschaffen. Bei einer weiteren Ausführungsform oder einem alternativen Modus kann die Stärke der Vibration auch von der an der Abspielvorrichtung eingestellten Lautstärke abhängig gesteuert werden.

Bei einer noch weiteren Ausführungsform kann, beispielsweise in einem Elektroauto, ein künstlich erzeugtes Motorengeräusch als akustisches Primärsignal, welches in diesem Falle ein Unterhaltungssignal ist, dienen. Unabhängig von einer Lautstärke des abgespielten Motorengeräuschs kann dieses zur Ansteuerung der Massageeinheiten genutzt werden. Somit können beispielsweise die Vibrationen eines Verbrennungsmotors imitiert werden. Ferner können die Filter für eine zusätzliche Musikwiedergabe an ein etwaiges Motorengeräusch angepasst werden.

Gemäß einer Ausführungsform ist die Stärke der durch die Massageeinheiten erzeugten Massage einstellbar vorgesehen. Insbesondere ist dazu die Stärke einer Vibration der Massageeinheiten einstellbar. Alternativ oder zusätzlich ist das verwendete Frequenzband einstellbar vorgesehen. Insbesondere ist dazu die Lage und Breite, d.h. die Art der verschiedenen Frequenzbereiche des akustischen Unterhaltungssignals, die zu einem Ausgangssignal gewandelt werden, einstellbar. Somit ist die Vibrationsfrequenz der Massageeinheiten nach Kunden- oder nach Bedienerwunsch einstellbar. Weiterhin alternativ oder zusätzlich ist die Verteilung des angesteuerten Teils der Massageeinheiten einstellbar vorgesehen. Insbesondere ist dazu die Lage und Anzahl der angesteuerten Massageeinheiten einstellbar. Es können auch mehrere Lagen von Massageeinheiten, beispielsweise Sitzfläche oder Lehnenfläche, davon jeweils einzeln die linke und rechte Seite, und jeweils die Anzahl der Massageeinheiten einstellbar sein. Vorzugsweise handelt es sich bei der Einstellbarkeit jeweils um eine freie Einstellbarkeit. Vorteilhaft sind somit vielfältigste Einstellmöglichkeiten zur individuellen Anpassung der Massage zur Verfügung gestellt.

Gemäß einer Ausführungsform wird das akustische Unterhaltungssignal für die Ansteuerung der Massageeinheiten, insbesondere durch Pulsweitenmodulation (PWM), bevorzugt mittels eines einzelnen High-Side-Schalters, in eine Folge von Pulsen gewandelt. Vorteilhaft wird somit, beispielsweise in Form eines sogenannten Class-D Verstärkers (PWM), ein besonders einfacher, sparsamer und preiswerter Verstärker zum Ansteuern der Massageeinheiten eingesetzt.

Gemäß einer Ausführungsform wird der Dynamikverlauf des akustischen Unterhaltungssignals für die Ansteuerung komprimiert oder expandiert. Ferner kann auch beides, insbesondere in Stufen, stattfinden. Das Komprimieren dient dabei insbesondere der Einschränkung des Dynamikumfangs des akustischen Unterhaltungssignals. Mit Hilfe der Expansion kann der Dynamikumfang des akustischen Unterhaltungssignals vergrößert werden. Beispielsweise ist dazu ein Dynamikprozessor, zur Komprimierung insbesondere ein Dynamikkompressor und/oder zur Expansion insbesondere ein Dynamikexpander, vorgesehen, welcher das primäre Unterhaltungssignal entsprechend bearbeitet. Insbesondere kann mittels eines Dynamikexpanders eine Absenkung der niedrigen Geräuschpegel als ein kontinuierlicher Effekt erzielt werden, während größere Pegel unbeeinflusst bleiben. Somit kann beispielsweise ein Rauschen von der Steuerung ausgeklammert werden. Ferner kann vorteilhaft mittels eines Dynamikkompressors ein hoher Geräuschpegel bzw. Pegelspitzen, also ein impulsartiger Effekt, abgeschwächt werden. Somit können vorteilhaft das Gesamtsignal verstärkt und dabei insbesondere niedrigere Geräuschpegel angehoben werden, ohne dass es zu einer Überbelastung der Massageeinheiten kommt.

Gemäß einer Ausführungsform wird das akustische Unterhaltungssignal mittels einer Audiosignalquelle, insbesondere einer mobilen Audiosignalquelle oder einer fahrzeuginternen Audiosignalquelle, in die Abspielvorrichtung eingespeist. Es kann sich auch um eine sitzintegrierte Audiosignalquelle oder um eine fahrzeugexterne Audiosignalquelle handeln. Die Abspielvorrichtung ist dazu mit der Audiosignalquelle gekoppelt. Im Fall einer mobilen Audiosignalquelle kann dies über ein Anschlusskabel oder per drahtloser Datenübertragung geschehen. Im Fall einer fahrzeuginternen Audiosignalquelle ist diese vorzugsweise in das Unterhaltungssystem des Fahrzeugs integriert und insbesondere fest damit gekoppelt. Bei einer fahrzeugexternen Audiosignalquelle kann es sich beispielsweise um einen digitalen oder analogen Radio- oder Fernsehsender handeln. In diesem Fall weist die Abspielvorrichtung ist eine entsprechende Empfangsvorrichtung, insbesondere einen sogenannten Tuner, auf.

Gemäß einer Ausführungsform werden die Massageeinheiten zur Vibration in Frequenzbereichen angesteuert, in welchen sie nach außen hin für andere als auf dem betroffenen Sitz sitzende Personen, insbesondere für andere Fahrzeuginsassen, lediglich unwahrnehmbare Geräuschemission verursachen. Insbesondere werden die Massageeinheiten dabei zur Vibration in Frequenzbereichen angesteuert, welche keine Resonanzfrequenz der Struktur des Fahrzeugsitzes darstellen. Vorzugsweise handelt es sich um Frequenzen, welche durch die Struktur des Fahrzeugsitzes in hohem Maße gedämpft werden. Somit wird durch die Vibration vorteilhaft kein oder nur unterhalb der Wahrnehmungsgrenze Schall ausgegeben. Es können auch gezielt gewisse Massageeinheiten nicht einbezogen werden oder angepasst, beispielsweise angepasst an eine Geräuschumgebung, nur zugeschaltet werden, wenn dies nicht zu Lasten des Geräuschkomforts geht. Ferner können an unterschiedlichen Massageeinheiten die Frequenzen der Vibration unterschiedlich angepasst sein. Beispielsweise können dazu in bestimmten Bereichen des Sitzes nur vorbestimmte Frequenzen ausgegeben werden. Insbesondere kann dazu die Drehzahl eines Unwuchtmotors auf vorbestimmte Bereiche eingegrenzt sein. Die Ausgabe der vorbestimmten Frequenzen kann an die lokale an dem jeweiligen Sitzbereich vorgesehene Dämpfung und/oder an das Gewicht einer auf dem Sitz sitzenden Person angepasst gewählt sein.

Gemäß einer Ausführungsform werden die Massageeinheiten derart angeordnet und angesteuert, dass ihre Vibration von einer elektromotorischen Sitzverstellung gleichzeitig erzeugte Verstellgeräusche überdeckt oder neutralisiert. Insbesondere können dazu einzelne oder verschiedene Massageeinheiten von der Lage her passend an eine Verstellebene gekoppelt vorgesehen sein, um so Verstellgeräusche zu überdecken.

Gemäß einer Ausführungsform kann die Steuerung der einzelnen Massageeinheiten abhängig von der Stellung von einzelnen Sitzkomponenten gewählt werden. Insbesondere handelt es sich um Sitzkomponenten, in welchen die betroffenen Massageeinheiten angeordnet sind. Beispielsweise kann die Steuerung abhängig von der Einstellung einer Lordosenstütze erfolgen, insbesondere mit einer geringeren Vibration bei ganz nach vorne ausgefahrener Lordosenstütze.

Gemäß einer Ausführungsform erfolgt parallel zur Ansteuerung der Massageeinheiten eine Lautsprecherausgabe des akustischen Unterhaltungssignals. Vorteilhaft kann so, insbesondere im Falle von Musik als Unterhaltungssignal, durch die akustischen Reize die mit den Massageeinheiten erzeugte Massage intensiver empfunden. Ferner wird das Hörerlebnis durch die vom Sitz übertragene Massage erweitert. Musik wird so über die in den Sitz integrierten Massageeinheiten von einem Insassen in Form einer Massage zusätzlich gespürt. Somit wird die Musik insgesamt intensiver empfunden. Da die akustischen und taktilen Reize aufeinander abgestimmt sind entsteht insgesamt vorteilhaft ein intensiveres Erlebnis, als wenn die Massage ohne eine Lautsprecherausgabe der Musik abläuft.

Gemäß einer Ausführungsform einer Sitzanordnung zur Durchführung eines erfindungsgemäßen Verfahrens sind die Massageeinheiten als in unterschiedlichen Frequenzbereichen betreibbare Unwuchtmotoren ausgebildet. Somit sind die Frequenzen vorteilhaft anpassbar. Insbesondere handelt es sich bei den Unwuchtmotoren um Unwucht-Schrittmotoren. Vorteilhaft können somit zusätzlich auch die Positionen des exzentrischen Gewichts der jeweiligen Massageeinheiten aufeinander abgestimmt werden. Insbesondere können so synchrone Vibrationsphasen oder bestimmte Ausbreitungsmuster der Vibration erzeugt werden. Vorteilhaft sind somit zusätzliche Funktionen geschaffen.

Gemäß einer Ausführungsform einer Sitzanordnung ist ein Gleichrichterelement in der Motorzuleitung oder in einer Verbindung zwischen der Abspielvorrichtung und der Steuervorrichtung angeordnet. Insbesondere kann es sich um eine Diode handeln. Diese kann beispielsweise in einer Audiozuleitung eines Verstärkers eingesetzt vorgesehen sein. Somit wird die Motorschwungmasse nicht aktiv durch eine negative Halbwelle gebremst. Vorteilhaft werden somit die Leistungsaufnahme der Motoren, insbesondere Unwuchtmotoren, der Massageeinheiten verringert und der Vibrationseffekt gesteigert. Insbesondere kann entweder ein Gleichrichterelement oder ein High-Side-Schalter vorgesehen sein.

Die obigen Ausgestaltungen und Weiterbildungen lassen sich, sofern sinnvoll, beliebig miteinander kombinieren. Insbesondere sind die Merkmale des Verfahrens zur Ansteuerung von Massageeinheiten in einem Sitz auf eine Steuervorrichtung zum Steuern von Massageeinheiten in einem Sitz übertragbar und/oder mit den Merkmalen einer Sitzanordnung zur Durchführung eines solchen Verfahrens kombinierbar, und umgekehrt.

### INHALTSANGABE DER ZEICHNUNG

Die vorliegende Erfindung wird nachfolgend anhand der in den schematischen Figuren der Zeichnung angegebenen Ausführungsbeispiele näher erläutert. Es zeigen dabei:
- Fig. 1: ein Ablaufdiagramm einer Ausführungsform des erfindungsgemäßen Verfahrens;
- Fig. 2: ein Blockschaltbild einer Ausführungsform der erfindungsgemäßen Sitzanordnung;
- Fig. 3: ein Ablaufdiagramm einer weiteren Ausführungsform des erfindungsgemäßen Verfahrens;
- Fig. 4: eine Massageeinheit gemäß einer ersten Ausführungsform;
- Fig. 5: eine Massageeinheit gemäß einer zweiten Ausführungsform;
- Fig. 6: eine Gruppe von Massageeinheiten gemäß der zweiten Ausführungsform;
- Fig. 7: die Massageeinheit gemäß Fig. 4 in einer Ausnehmung eines Polsters;
- Fig. 8: die Massageeinheiten gemäß Fig. 5 in einer Ausnehmung eines Polsters;
- Fig. 9: ein Fahrzeugsitz mit einer Massagevorrichtung nach einer ersten Ausführungsform;
- Fig. 10: ein Fahrzeugsitz mit einer Massagevorrichtung nach einer zweiten Ausführungsform;
- Fig. 11: ein Fahrzeugsitz mit einer Massagevorrichtung, welche eine Vielzahl von Massageeinheiten gemäß Fig. 4 aufweist;
- Fig. 12: eine schematische Darstellung von einer in einem Fahrzeugsitz integrierten Massagevorrichtung, welche formkodierte Massageeinheiten aufweist;
- Fig. 13: ein Polster eines Fahrzeugsitzes mit formkodierten Ausnehmungen;
- Fig. 14: eine schematische Darstellung eines in ein Polster eines Fahrzeugsitzes eingeschäumten Verbindungselements;
- Fig. 15: eine Längsschnittansicht eines Fahrzeugsitzes mit einer Massagevorrichtung, welche eine Gruppe von Massageeinheiten gemäß Fig. 6 aufweist;

- Fig. 16: eine Ausführungsform eines Gehäuses für eine Massageeinheit.

Die beiliegenden Figuren der Zeichnung sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts anderes ausgeführt ist - jeweils mit denselben Bezugszeichen versehen.

### BESCHREIBUNG VON AUSFÜHRUNGSBEISPIELEN

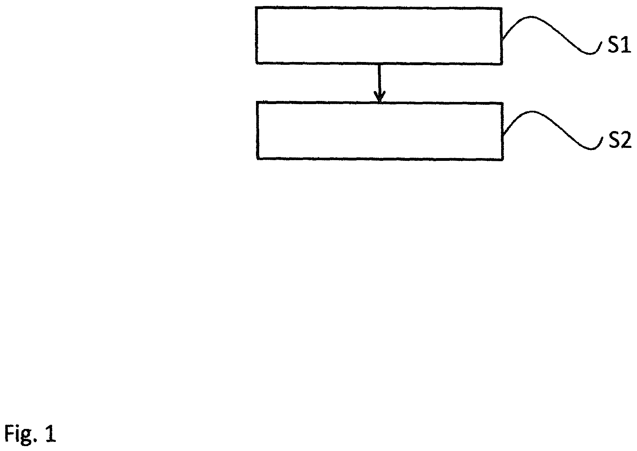

Fig. 1 zeigt ein Ablaufdiagramm einer Ausführungsform des erfindungsgemäßen Verfahrens zur Ansteuerung von Massageeinheiten 102-1 bis 102-n in einem Sitz 103. Der Sitz 103 kann dabei z.B. insbesondere ein Fahrzeugsitz 103 sein.

Das Verfahren sieht das Abspielen, Sl, eines akustischen Unterhaltungssignals 104 vor. Das Verfahren sieht ferner das Steuern S2 von in den Sitz 103 integrierten Massageeinheiten 102-1 bis 102-n basierend auf dem akustischen Unterhaltungssignal 104 vor.

Das akustische Unterhaltungssignal 104 kann z.B. ein Musikstück sein, welches mittels einer Abspielvorrichtung 105 abgespielt wird. Insbesondere bei einem Fahrzeugsitz 103, also der Anwendung des Verfahrens in einem Fahrzeug, kann die Abspielvorrichtung 105 ein Unterhaltungssystem, z.B. ein Radio oder CD-Spieler, des jeweiligen Fahrzeugs sein oder mit diesen gekoppelt sein.

Die Massageeinheiten 102-1 bis 102-n können über die Sitzfläche des Sitzes 103 verteilt angeordnet sein. Dabei können zur Ansteuerung von an unterschiedlichen Stellen angeordneten Massageeinheiten 102-1 bis 102-n verschiedene Frequenzbereiche des akustischen Unterhaltungssignals 104 als Ausgangssignal 107 ausgegeben werden. Beispielsweise können höhere Frequenzbereiche des akustischen Unterhaltungssignals 104 genutzt werden, um Massageeinheiten 102-1 bis 102-n im Schulter- und Nackenbereich anzusteuern, während tiefe Frequenzbereiche des akustischen Unterhaltungssignals 104 genutzt werden können, um Massageeinheiten 102-1 bis 102-n im Bein- und Gesäßbereich anzusteuern. Es versteht sich, dass diese Aufteilung lediglich beispielhaft ist und weitere Varianten möglich sind.

Die Aufteilung der Frequenzen kann dabei nicht nur nach deren Höhe erfolgen. Beispielsweise können die Massageeinheiten 102-1 bis 102-n zur Vibration in Frequenzbereichen angesteuert werden, in welchen sie nach außen hin für andere als auf dem betroffenen Sitz 103 sitzende Personen, insbesondere für andere Fahrzeuginsassen, lediglich unwahrnehmbare Geräuschemission verursachen. Zusätzlich oder alternativ können die Massageeinheiten 102-1 bis 102-n derart angeordnet und angesteuert werden, dass ihre Vibration bzw. das dadurch hervorgerufene Geräusch Verstellgeräusche überdeckt oder neutralisiert, die von einer elektromotorischen Sitzverstellung erzeugt werden.

Ferner kann beim Steuern S2 lediglich ein Teil der in den Sitz 103 integrierten Massageeinheiten 102-1 bis 102-n mit dem auf dem Unterhaltungssignal 104 basierenden Ausgangssignal 107 angesteuert werden. So kann beispielsweise ein Benutzer des Sitzes 103 selbst die Bereiche des Rückens bzw. der Beine wählen, welche durch die Massageeinheiten 102-1 bis 102-n massiert werden sollen. Ferner kann eine Mehrkanal-Musikwidergabe, beispielsweise Stereo oder 5.1, mit dem auf dem Unterhaltungssignal 104 basierenden Ausgangssignal 107 abgebildet werden.

Vorzugsweise wird lediglich ein Teil der in den Sitz 10 integrierten Massageeinheiten 102-1 bis 102-n mit dem auf dem Unterhaltungssignal 104 basierenden Ausgangssignal 107 gleichzeitig angesteuert. Eine Steuervorrichtung 106 verteilt dazu die Pegel zur Ansteuerung der Massageeinheiten 102-1 bis 102-n derart, dass nicht alle in dem Sitz 103 vorgesehenen Massageeinheiten 102-1 bis 102-n gleichzeitig auf einen Pegel reagieren. Stattdessen verteilt die Steuereinrichtung 106 die Pegel derart, dass sich die Massageeinheiten 102-1 bis 102-n abwechseln und ihre Motoren so für eine vorbestimmte zweite Zeitdauer beispielsweise zur Abkühlung angehalten werden können. Vorzugsweise werden die Abkühlphasen einzelner Massageeinheiten 102-1 bis 102-n harmonisch in den Massageablauf integriert, so dass die Abwechslung der Massageeinheiten 102-1 bis 102-n für den Insassen nicht wahrnehmbar bzw. unspürbar ist.

Schließlich kann das akustische Unterhaltungssignal 104 insbesondere unabhängig von einer an der Abspielvorrichtung 105 eingestellten Ausgabelautstärke als Eingangsgröße für das Ansteuern verwendet werden. Mit einer derartigen Ausführung ist beispielsweise Liederraten anhand der Massage möglich, wenn die Ausgabelautstärke auf Null eingestellt ist. Selbstverständlich kann das akustische Unterhaltungssignal 104 aber auch in Abhängigkeit der Ausgabelautstärke der Abspielvorrichtung 105 angepasst werden. Die Ausgabelautstärke bestimmt in solch einer Ausführung folglich die Stärke der Vibrationen.

In einer Ausführungsform kann eine Diode in den Signalpfad, also in einer Verbindung zwischen der Abspielvorrichtung 105 und der Steuervorrichtung 106, angeordnet sein. Die Diode dient dabei als Gleichrichtervorrichtung, sodass nur positive Signalanteile an die Massageeinheiten 102-1 und 102-2 übertragen werden.

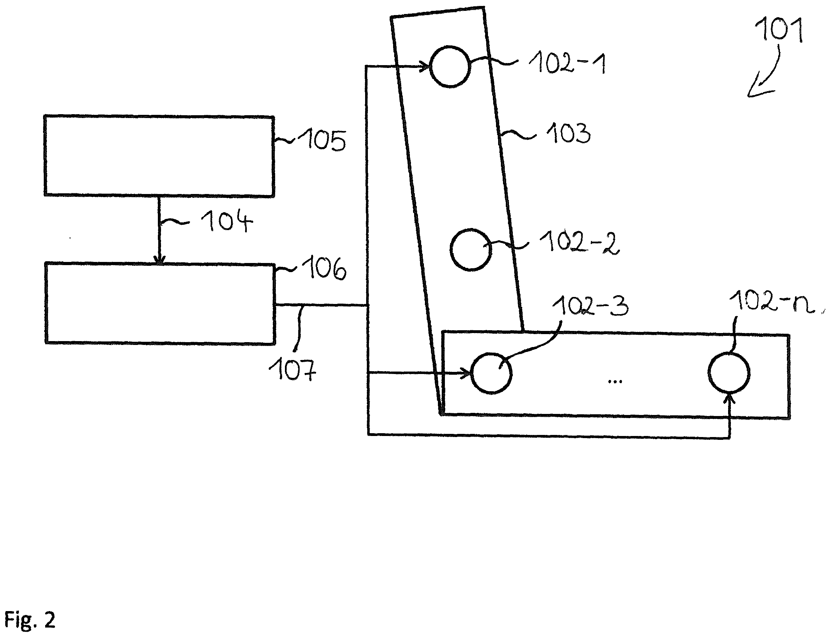

Fig. 2 zeigt ein Blockschaltbild einer Ausführungsform der erfindungsgemäßen Sitzanordnung 101.

In dem Sitz 103 ist eine Anzahl, also eine oder mehrere, von Massageeinheiten 102-1 bis 102-n, hier konkret vier, angeordnet. Dabei sind in Fig. 2 lediglich beispielhaft die Massageeinheiten 102-1 und 102-2 in der Sitzlehne und die Massageeinheiten 102-3 und 102-n in der Sitzfläche angeordnet. Weitere Massageeinheiten sind durch drei Punkte angedeutet. Die Anzahl und Anordnung der Massageeinheiten kann je nach Anwendung variiert werden.

Zur Ansteuerung der Massageeinheiten 102-1 bis 102-n ist eine Steuervorrichtung 106 vorgesehen, die aus einem von einer Abspielvorrichtung 105 bereitgestellten Unterhaltungssignal 104 ein Steuersignal 107 für die Massageeinheiten 102-1 bis 102-n erzeugt und an diese weiterleitet.

Das Unterhaltungssignal 104 kann z.B. ein Audiosignal sein, welches ein Musikstück oder dergleichen darstellt. Dabei kann das Unterhaltungssignal 104 aus unterschiedlichen Quellen stammen. Beispielsweise kann die Abspielvorrichtung 105 ein in einem Fahrzeug installiertes Audio-System mit einem Radio oder CD-Player sein. Die Abspielvorrichtung 105 kann aber z.B. auch das Audio-System sein, welches über Bluetooth von z.B. einem Smartphone das Unterhaltungssignal 104 empfängt.

Insbesondere können auch die Steuervorrichtung 106 und die Abspielvorrichtung 105 in einem einzigen Gerät zusammengefasst bzw. integriert sein. Erfindungsgemäß ist in dem Fahrzeug-Audio-System die Steuervorrichtung 106 integriert. Die Massageeinheiten 102-1 bis 102-n sind in einer Ausführungsform direkt mit der Abspielvorrichtung 105 gekoppelt, welche die notwendige Ansteuerschaltung zum Bereitstellen des Ausgangssignals 107 aufweist. Sowohl die Abspielvorrichtung 105 als auch die Steuervorrichtung 106 können dabei zumindest teilweise als Computerprogrammmodule ausgebildet sein, die von einer Recheneinrichtung des Audio-Systems ausgeführt werden.

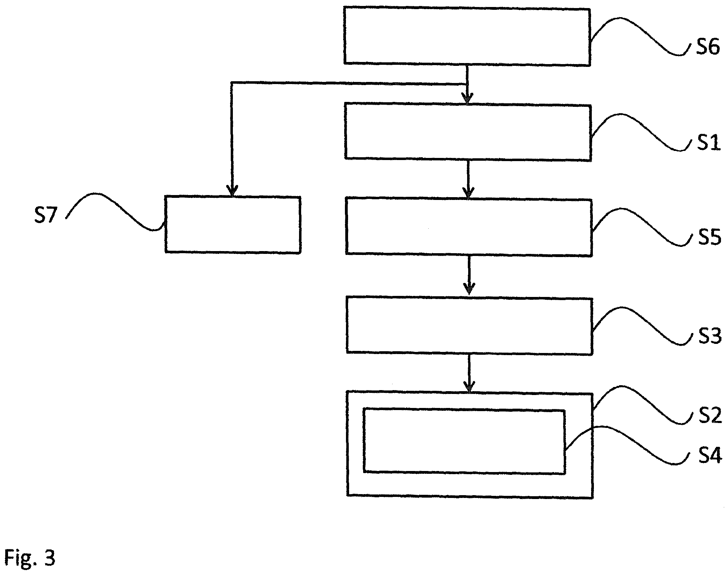

Fig. 3 zeigt ein Ablaufdiagramm einer weiteren Ausführungsform des erfindungsgemäßen Verfahrens. Das Verfahren der Fig. 3 basiert auf dem Verfahren der Fig. 1 und weist weitere Schritte S3 bis S7 auf. Die Nummerierung der einzelnen Schritte S1 - S7 dient lediglich der Differenzierung und impliziert keinerlei Reihenfolge. Insbesondere können einzelne Schritte auch an anderer Position durchgeführt werden, als dies im Folgenden dargestellt wird.

Der Schritt S6 vor dem Schritt S1 sieht vor, dass das akustische Unterhaltungssignal 104 mittels einer Audiosignalquelle in die Abspielvorrichtung 105 eingespeist wird. Die Audiosignalquelle kann dabei insbesondere eine mobile Audiosignalquelle, wie z.B. ein Smartphone oder dergleichen, oder eine fahrzeuginterne Audiosignalquelle, wie z.B. ein CD-Spieler sein.

Nach dem Abspielen S1 des akustischen Unterhaltungssignals 104 kann der Dynamikverlauf des akustischen Unterhaltungssignals 104 für die Ansteuerung der Massageeinheiten 102-1 bis 102-n komprimiert und/oder expandiert werden, S5. Das Komprimieren dient dabei insbesondere der Einschränkung des Dynamikumfangs des akustischen Unterhaltungssignals 104, wobei mit Hilfe der Expansion der Dynamikumfang des akustischen Unterhaltungssignals 104 vergrößert werden kann. Nach Schritt S5 werden in Schritt S4 verschiedene Frequenzbereiche des akustischen Unterhaltungssignals 104 jeweils zu einem Ausgangssignal 107 gewandelt. Beispielsweise können unterschiedliche Massageeinheiten 102-1 bis 102-n mit Signalen unterschiedlicher Frequenz angesteuert werden. Es kann auch vorgesehen sein, dem Benutzer die Auswahl der Frequenzen oder der mit den jeweiligen Frequenzen anzusteuernden Massageeinheiten 102-1 bis 102-n zu ermöglichen. Insbesondere in einem Fahrzeug kann z.B. in der Head-Unit, also einer zentralen Anzeige- und Steuereinheit, eine Art Equalizer angezeigt werden, den der Benutzer einstellen kann. Die Massageeinheiten 102-1 bis 102-n können dazu insbesondere auch in Gruppen zusammengefasst angesteuert werden.

Die Ansteuerung der Massageeinheiten 102-1 bis 102-n kann insbesondere mittels eines pulsweitenmodulierten Signals erfolgen. Dazu kann in Schritt S4 das akustische Unterhaltungssignal 104 z.B. mittels eines einzelnen High-Side-Schalters in eine Folge von entsprechenden Pulsen gewandelt werden.

Parallel zur Ansteuerung der Massageeinheiten 102-1 bis 102-n kann eine Lautsprecherausgabe des akustischen Unterhaltungssignals 104 erfolgen, S7.

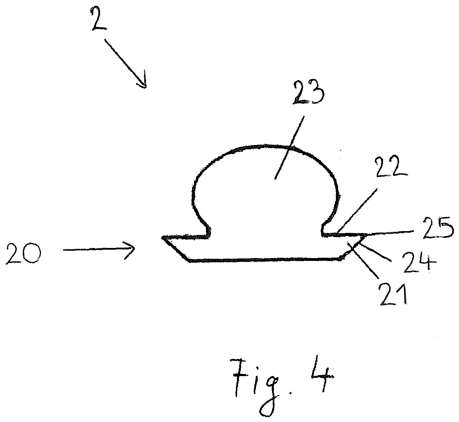

Fig. 4 zeigt eine Massageeinheit 2 gemäß einer ersten Ausführungsform. Diese kann bei dem in Bezug auf Fig. 1 bis 3 beschriebenen Verfahren und/oder Fahrzeugsitz verwendet werden.

Die Massageeinheit 2 weist eine sich bei Einsetzen der Massageeinheit 2 in eine Ausnehmung eines Polster selbstbefestigende Ausbildung 20 auf.

Bei der vorliegenden Ausführungsform ist die selbstbefestigende Ausbildung 20 mit Widerhaken 21 realisiert, welche integral mit einem Gehäuse 22 der Massageeinheit 2 gebildet sind.

Die Widerhaken 21 können umlaufend oder abschnittsweise an dem Gehäuse 22 vorgesehen sein. Sie weisen eine schräge Stirnfläche 24 auf, welche seitlich über das restliche Gehäuse 22 übersteht. Die Widerhaken 21 weisen ferner an ihrer Rückseite eine scharfe Kante 25 auf.

Die Massageeinheiten 2 weist ferner einen innerhalb des Gehäuses 22 angeordneten, zur besseren Übersichtlichkeit nicht im Einzelnen dargestellten Vibrationsgenerator 23 auf. Der Vibrationsgenerator 23 dient als Massage-Aktor und ist beispielsweise als Unwuchtmotor ausgebildet. Bei dem Unwuchtmotor wird ein auf der Ankerwelle eines Elektromotors außermittig zur Drehachse des Elektromotors angeordnetes Gewicht genutzt. Durch Drehung des Motors entsteht damit eine Vibration. Sofern eine Massageeinheit 2 Teil einer Massagevorrichtung eines Fahrzeugsitzes ist, kann eine derartige Vibration beispielsweise durch einen Bezug des Fahrzeugsitzes hindurch zur Massage auf einen Insassen übertragen werden.

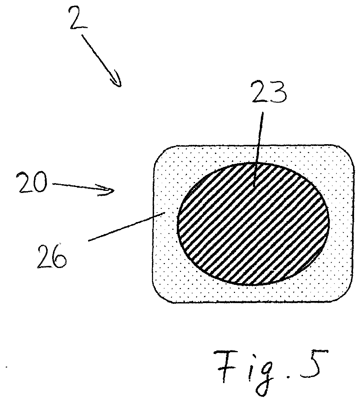

Fig. 5 zeigt eine Massageeinheit 2 gemäß einer zweiten Ausführungsform. Diese kann bei dem in Bezug auf Fig. 1 bis 3 beschriebenen Verfahren und/oder Fahrzeugsitz ebenso verwendet werden.

Bei dieser Ausführungsform umfasst die selbstbefestigende Ausbildung 20 eine Einbettung der Massageeinheit in ein Schaumkissen 26.

Der Schaum des Schaumkissens 26 weist in Reibpaarung mit einem Polster eines Fahrzeugsitzes einen sehr hohen Reibungskoeffizienten auf. Aufgrund der hohen Reibung kann eine Selbstbefestigung der Massageeinheit stattfinden, wenn das Schaumkissen 26 bzw. die Massageeinheit 2 mit dem Schaumkissen 26 in eine Ausnehmung eines Polsters eingeführt wird.

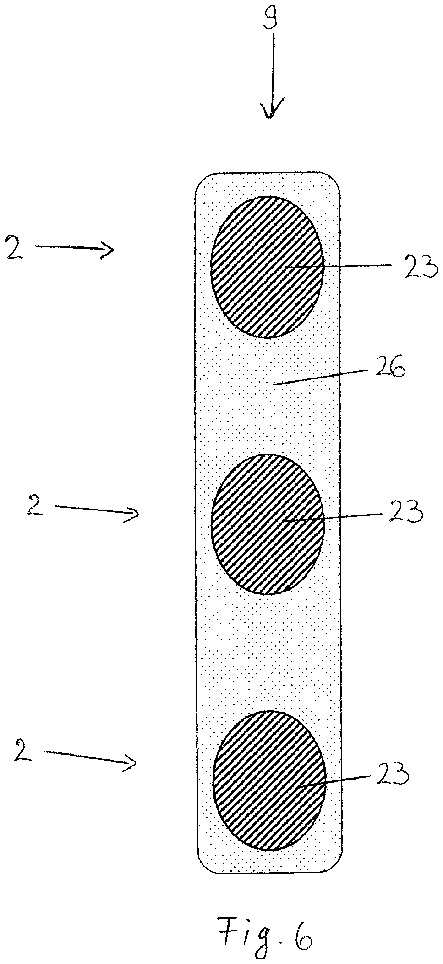

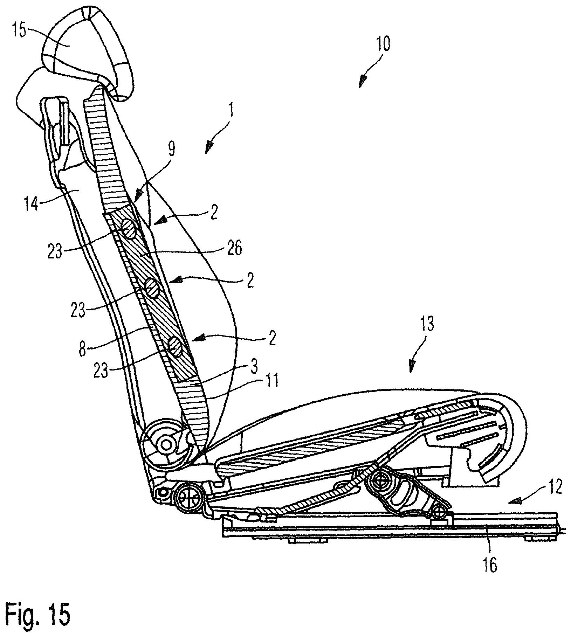

Fig. 6 zeigt eine Gruppe 9 von Massageeinheiten 2 gemäß der zweiten Ausführungsform. Es handelt sich dabei um eine Gruppe 9 von drei in einer Reihe in ein gemeinsames Schaumkissen 26 eingebetteten bzw. eingeschäumten Massageeinheiten 2. Jede Massageeinheit 2 weist dabei einen Vibrationsgenerator 23 auf. Eine derartige Gruppe 9 kann gemeinsam in ein Polster eines Fahrzeugsitzes integriert werden, wie in Bezug auf Fig. 15 näher erläutert.

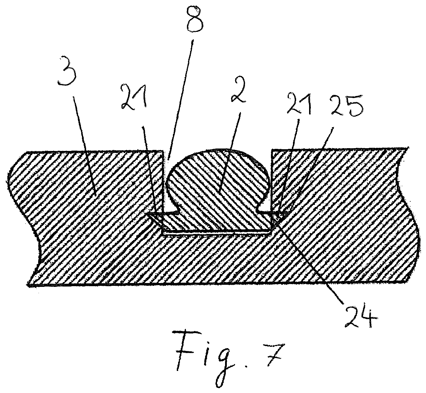

Fig. 7 zeigt die Massageeinheit gemäß Fig. 4 in einer Ausnehmung 8 eines Polsters 3.

Das Polster 3 kann insbesondere ein Polster eines Fahrzeugsitzes darstellen.

Die schräge Stirnfläche 24 der Widerhaken 21 gleitet beim Einführen der Massageeinheit 2 in die Ausnehmung 8 des Polsters 3 am Material des Polsters 3 entlang, sodass das Polster 3 dabei lokal elastisch verformt wird. Wenn die Kante 25, welche den äußersten Abschnitt des Widerhakens 21 bildet, das Polster 3 passiert, dehnt sich das Polster 3 dahinter wieder aus. Die Kante 25 des Widerhakens 21 gräbt sich daher im Falle einer Bewegung der Massageeinheit 2 aus der Ausnehmung heraus in das Polster 3 ein. Somit ist die einmal in die Ausnehmung 8 eingeführte Massageeinheit 2 darin selbsttätig befestigt bzw. selbstbefestigt.

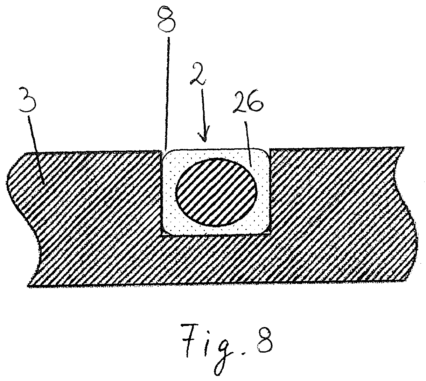

Fig. 8 zeigt die Massageeinheit 2 gemäß Fig. 5 in einer Ausnehmung 8 eines Polsters 3.

Das Polster 3 kann insbesondere ein Polster eines Fahrzeugsitzes darstellen.

Aufgrund des hohen Reibungskoeffizienten zwischen dem Schaum des Schaumkissens 26 und dem Polster 3 liegt eine Selbstbefestigung vor, wenn die Massageeinheit 2 mit dem Schaumkissen 26 in die Ausnehmung 8 des Polsters 3, wie dargestellt, eingeführt ist.

Die Reibungskraft und damit die Selbstbefestigung kann verstärkt werden, indem die Größe des Schaumkissens 26 mit einem Übermaß auf die Ausnehmung 8 abgestimmt ist. Es liegt dann eine gewisse Pressung zwischen Schaumkissen 26 und Ausnehmung 8 vor, sodass das Schaumkissen 26 verstärkt in der Ausnehmung 8 haftet. Zur Montage wird der Schaum des Schaumkissens 26 mittels eines entsprechenden Werkzeugs, beispielsweise einer entsprechend geformten Zange, elastisch komprimiert. Nach dem Einführen in die Ausnehmung 8 und nach der Freigabe des Werkzeugs dehnt sich der Schaum des Schaumkissens 26 elastisch aus, sodass eine Selbstbefestigung der Massageeinheit 2 in der Ausnehmung 8 erfolgt.

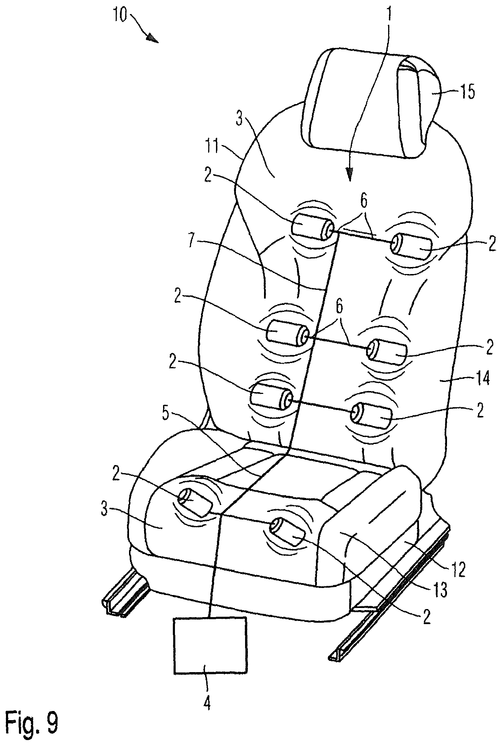

Fig. 9 zeigt einen Fahrzeugsitz 10 mit einer ersten Ausführungsform einer Massagevorrichtung 1.

Der Fahrzeugsitz 10 weist eine in Führungsschienen 16 laufende Basis 12, eine Sitzfläche 13, eine Rückenlehne 14 und eine Kopfstütze 15 auf. Zusätzlich können nicht dargestellte Armlehnen vorgesehen sein.

Die Massagevorrichtung 1 ist in den Fahrzeugsitz 10 integriert vorgesehen. Sie weist eine Vielzahl von Massageeinheiten 2 auf, welche in das Polster 3 des Fahrzeugsitzes 10 integriert sind. In der vorliegenden Ausführungsform weisen sowohl die Sitzfläche 13 als auch die Rückenlehne 14 ein solches Polster 3 auf.

Darüber hinaus ist eine Steuervorrichtung 4 vorgesehen, welche zum Ansteuern sämtlicher Massageeinheiten 2 vorgesehen und ausgebildet ist. Beispielsweise kann es sich dabei um eine Rechenvorrichtung mit einem entsprechenden Computerprogramm bzw. ein Steuergerät handeln, welches die einzelnen Massageeinheiten mit geeigneten Steuersignalen versorgt. Insbesondere können auch die Steuervorrichtung 4 und eine Abspielvorrichtung in einem einzigen Gerät zusammengefasst bzw. integriert sein. Erfindungsgemäß ist in dem Fahrzeug-Audio-System die Steuervorrichtung 4 integriert. Die Steuervorrichtung 4 kann dabei zumindest teilweise als Computerprogrammmodul ausgebildet sein, das von einer Recheneinrichtung des Audio-Systems ausgeführt wird.

Die Steuervorrichtung 4 kann ferner mit einer Mensch-Maschine-Schnittstelle, beispielsweise einer zusätzlichen Knopftafel, einem Touchscreen-Bedienmodul oder mit der Bedieneinheit eines Unterhaltungssystems eines Fahrzeugs, gekoppelt sein, über welche ein Insasse die Massagevorrichtung 1 bedienen kann.

Ferner ist eine Verbindungseinrichtung 5 vorgesehen, welche die Steuervorrichtung 4 mit den Massageeinheiten 2 koppelt. Dazu weist die Verbindungseinrichtung 5 einen Kabelstrang 7 auf, in welchem für jede Massageeinheit 2 zumindest ein Kabel 6 gebündelt ist. Die Kabel 6 zweigen an den jeweiligen Stellen, insbesondere auf Höhe der ihnen zugeordneten Massageeinheiten 2, von dem Kabelstrang 7 ab und sind im montierten Zustand mit der jeweiligen Massageeinheit 2 kontaktiert.

Der Kabelstrang 7 verläuft nicht mittig, sondern seitlich bzw. asymmetrisch im Polster 3. Ferner weisen die jeweils auf einer gemeinsamen Höhe abzweigenden Kabel 6 unterschiedliche Längen auf. Somit ist die Verbindungseinrichtung 5 asymmetrisch ausgebildet.

Demnach ist der vorbestimmten Position jeder Massageeinheit 2 ein vorbestimmtes Kabel 6 zugeordnet, welches mit einer individuellen Kabellänge ausgestattet ist, so dass es nur zu dieser vorbestimmten Position der jeweiligen Massageeinheit 2, insbesondere ohne ein Kürzen oder Verlängern des Kabels, verlegt werden kann. Wenn ein Kabel 6 zu einer ihm nicht zugeordneten anderen Position einer anderen Massageeinheit 2 verlegt würde, wäre es zu kurz oder zu lang und somit zu der anderen Position nicht passend. In einem solchen Fall würde die Falschpositionierung einem das Kabel 6 verlegenden Werker durch die nicht passsende Kabellänge angezeigt.

Die Kontaktierung des Kabelstrangs 7 mit der Steuervorrichtung 4 ist zentral vorgesehen. Hierzu kann beispielsweise ein asymmetrischer Stecker mit einer vorbestimmten Pin-Belegung vorgesehen sein, welcher die für die einzelnen Massageeinheiten 2 vorbestimmten Kabel 6 auf ebenso vorbestimmte Pins legt. Die asymmetrische Anordnung der Kabel 6 ist somit auch beim Anschluss der Steuervorrichtung 4 mittels des asymmetrischen Steckers abgebildet. Die Steuervorrichtung 4 weist dementsprechend einen korrespondierenden asymmetrischen Gegenstecker auf. Somit ist das richtige Kontaktieren bzw. Anstecken des Kabelstrangs 7 an die Steuervorrichtung 4 gewährleistet.

Entsprechend der asymmetrischen bzw. nicht mittigen Anordnung des Kabelstrangs 7 ist auch die Steuervorrichtung 4 asymmetrisch bzw. nicht mittig am Fahrzeugsitz 10 angeordnet.

Im Gegensatz zur Verbindungseinrichtung 5 sind die Massageeinheiten 2 symmetrisch am jeweiligen Polster 3 verteilt integriert. Jeder Massageeinheit 2 ist dabei eine individuelle Kabellänge zugeordnet. Diese ist durch die Position bzw. Anordnung einer der jeweiligen Massageeinheit 2 zugehörigen Ausnehmung 8 vorgegeben.

Bei den Massageeinheiten 2 handelt es sich in der dargestellten Ausführungsform um einzelne Massageeinheiten 2. Die Steuervorrichtung 4 steuert die Drehzahl der Unwuchtmotoren der Massageeinheiten 2 und somit deren Vibrationsfrequenz. Die Steuervorrichtung 4 kann dementsprechend ein Massageprogramm abspielen, bei welchem die unterschiedlichen Massageeinheiten 2 zur Massage entsprechend angesteuert werden. Dies kann insbesondere gemäß einem in Bezug auf die Fig. 1 bis 3 beschriebenen Verfahren erfolgen.

In der dargestellten Ausführungsform ist die Steuervorrichtung außerhalb des Fahrzeugsitzes 10 angeordnet. Dies ist jedoch rein schematisch zu verstehen. Bei weiteren Ausführungen kann die Steuervorrichtung 4 auch in den Fahrzeugsitz 10, beispielsweise in dessen Basis 12 oder in das Polster 3, integriert sein.

Zur Montage der Massagevorrichtung 1 in den Fahrzeugsitz 10 werden die Massageeinheiten 2 mit den ihnen zugeordneten Kabeln 6 der Verbindungseinrichtung 5 kontaktiert und in die ihnen jeweils zugeordnete Ausnehmung 8 eingesetzt. Dabei kann entweder zuerst eine Kontaktierung und dann das Einsetzen erfolgen oder zuerst das Einsetzen und dann die Kontaktierung erfolgen. Das Einsetzen und Kontaktieren der Massageeinheiten 2 erfolgt hier jeweils einzeln.

In einem folgenden Fertigungsschritt wird das Polster 3 mit dem Bezug 11 überzogen. Die Massageeinheiten 2 sind somit im montierten Zustand, insbesondere komplett, verpolstert.

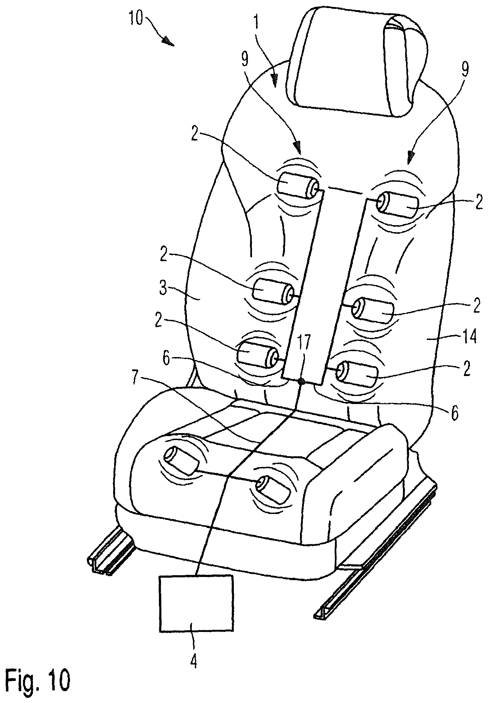

Fig. 10 zeigt ein Fahrzeugsitz mit einer Massagevorrichtung nach einer zweiten Ausführungsform.

Im Unterschied zur ersten Ausführungsform der Massagevorrichtung 1 gemäß Fig. 9 sind bei dieser zweiten Ausführungsform die Massageeinheiten 2 in Gruppen 9 angeordnet und kontaktiert bzw. verkabelt. Die Gruppen 9 sind dazu bevor der Montage der Massageeinheiten 2 vorkonfektioniert bzw. vorverkabelt ausgebildet. Bei der dargestellten Ausführungsform bedeutet dies, dass eine erste Gruppe 9 von drei Massageeinheiten 2 zur Integration in die linke Seite des Polsters 3 der Rückenlehne 14 bereits vor der Montage mit einem gemeinsamen Anschlusskabel 6 kontaktiert ist. Dieses gemeinsame Anschlusskabel 6 wird bei der Montage vor oder nach dem Einsetzen der Massageeinheiten 2 zur Mitte des Fahrzeugsitzes 10 geführt und dort mit dem Kabelstrang 7 der Verbindungseinrichtung 5 gekoppelt. In analoger Weise wird eine zweite Gruppe 9 von drei zur Integration in die rechte Seite des Polsters 3 der Rückenlehne 14 vorgesehenen Massageeinheiten 2 an der rechten Seite des Polsters 3 eingesetzt und mit einem gemeinsamen Anschlusskabel 6 dem Kabelstrang 7 der Verbindungseinrichtung 5 gekoppelt.

Die jeweiligen drei Massageeinheiten 2 der linken und rechten Gruppe 9 sind jeweils zur Montage auf einer vorbestimmten Höhe der Rückenlehne 14 vorbestimmt. Sie sind mit einer zu dieser Höhe der vorbestimmten Montage korrespondierenden Kabellänge ausgestattet, sodass ausgehend von einem Anschlusspunkt 17 der gemeinsamen Anschlusskabel 6 an der jeweiligen Seite des Polsters 3 nur eine zu der jeweiligen Kabellänge korrespondierende Ausnehmung im Polster 3 existiert. Somit kann die Gruppe 9 nur in der vorbestimmten Weise verbaut und mit dem Kabelstrang 7 kontaktiert werden. Einem Falschverbau ist somit wirksam vorgebeugt.

Am Anschlusspunkt 17 sowie an den Anschlusskabeln 6 sind asymmetrische Stecker vorgesehen sein, welche ausschließlich in der vorbestimmten Kontaktierung miteinander verbindbar sind. Somit ist eine korrekte Kontaktierung der Anschlusskabel 6 mit dem Kabelstrang 7 gewährleistet.

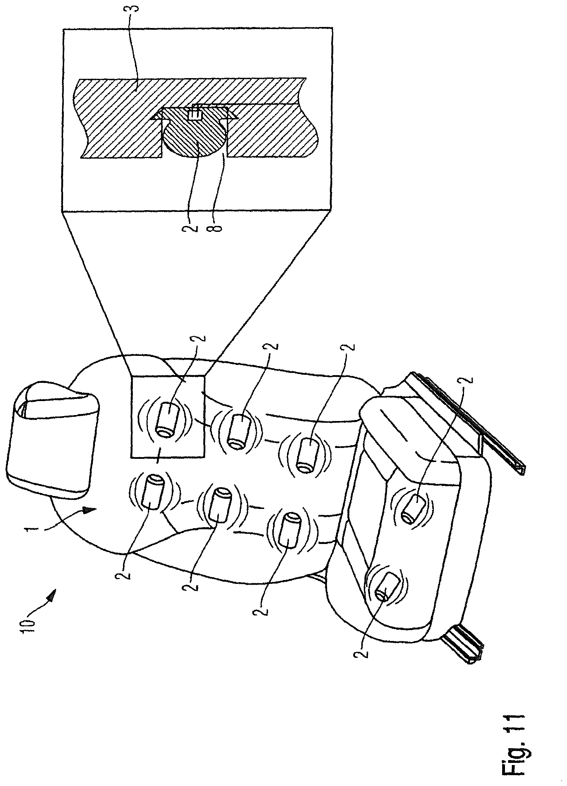

Fig. 11 zeigt einen Fahrzeugsitz 10 mit einer Massagevorrichtung 1, welche eine Vielzahl von Massageeinheiten 2 gemäß Fig. 4 aufweist. Die Massageeinheiten 2 sind in der in Bezug auf Fig.7 erläuterten Weise an vorbestimmten Stellen des Polsters 3 in Ausnehmungen 8 des Polsters 3 eingesetzt.

Es kann sich dabei um die erste Ausführungsform einer Massagevorrichtung 1 nach Fig. 9 mit einzelnen Massageeinheiten 2 oder um die zweite Ausführungsform einer Massagevorrichtung 1 nach Fig. 10 mit in Gruppen 9 vorkonfektionierten und/oder vorverkabelten Massageeinheiten 2 handeln.

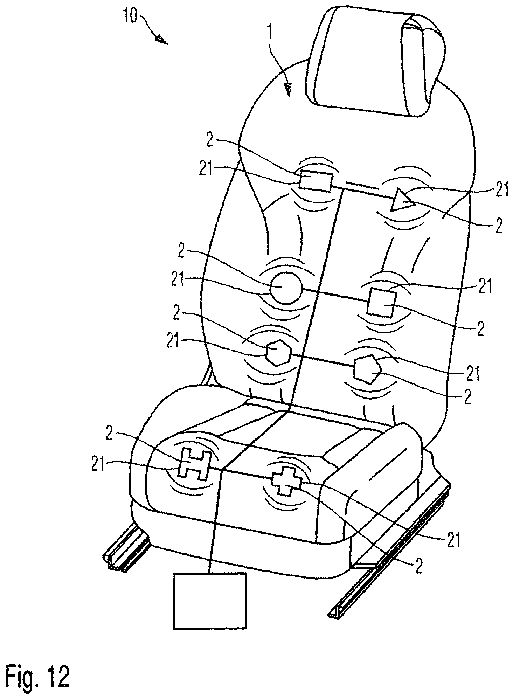

Fig. 12 zeigt eine schematische Darstellung von einer in einem Fahrzeugsitz 10 integrierten Massagevorrichtung 1, welche formkodierte Massageeinheiten 2 aufweist.

Jede Massageeinheit 2 weist dazu ein Gehäuse 21 mit einer individuellen Formgebung auf. Jede der formkodierten Massageeinheiten 2 ist dementsprechend dazu ausgelegt und vorgesehen, in eine entsprechend formkodierte Ausnehmung 8 des Polsters 3 des Fahrzeugsitzes 10 eingesetzt zu werden.

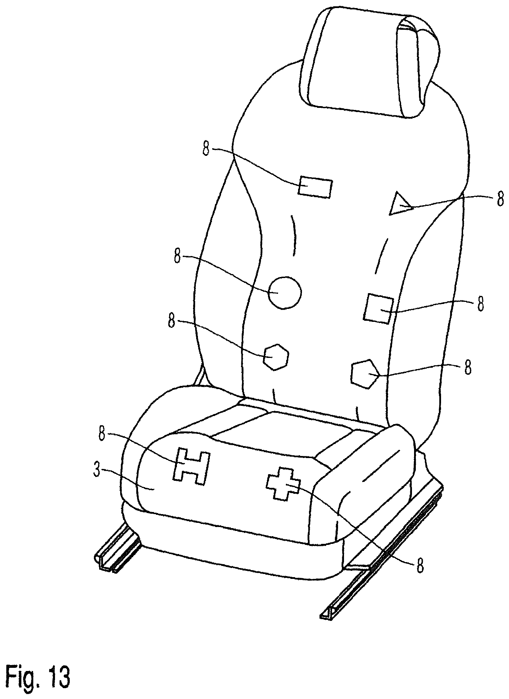

Fig. 13 zeigt schematisch ein Polster 3 eines Fahrzeugsitzes 10 mit derartig formkodierten Ausnehmungen 8.

Dabei weist jeweils nur eine Ausnehmung 8 innerhalb des Polsters 3 eine zu der individuellen Form eines Gehäuses 21 einer formkodierten Massageeinheit 2 korrespondierende Form auf. Somit ist sichergestellt, dass nur zueinander korrespondierende Massageeinheiten 2 und Ausnehmungen 8 miteinander verbaut werden können. Es wird daher bei der Montage die Einhaltung einer vorbestimmten Positionierung der formkodierten Massageeinheiten 2 in dem Polster 3 sichergestellt.

In analoger Weise können anstatt oder zusätzlich zu korrespondierenden Formen auch korrespondierende Farbkodierungen, d. h. Einfärbungen von Gehäusen 22 der Massageeinheiten 2 und von Ausnehmungen 8 des Polsters 3, vorgesehen sein. Die Farbkodierung kann auch auf die Kabel 6 der Verbindungseinrichtung 5 ausgedehnt werden, sodass beispielsweise unterschiedlich farbige Kabel 6 und entsprechend korrespondieren eingefärbte Schlitze im Polster 3 zu deren Verlegung vorgesehen sind.

Eine derartige Form und/oder Farbkodierung ist insbesondere dann sinnvoll, wenn für unterschiedliche Positionen am Polster 3, beispielsweise für unterschiedliche Höhen an der Rückenlehne 14 und/oder für Sitzpolster 13 und Rückenlehne 14 und/oder für die linke und rechte Seite, unterschiedliche Massageeinheiten 2 vorgesehen sind.

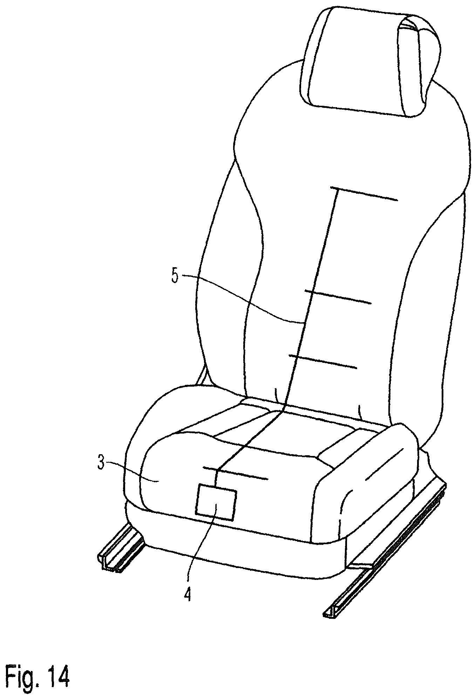

Fig. 14 zeigt eine schematische Darstellung eines in ein Polster 3 eines Fahrzeugsitzes 10 eingeschäumten Verbindungselements 5.

In der dargestellten Ausführungsform ist zusätzlich zur Verbindungseinrichtung 5 auch die Steuervorrichtung 4 in das Polster 3 eingeschäumt vorgesehen. Somit bilden das Polster 3, die Verbindungseinrichtung 5 und Steuervorrichtung 4 ein vorgefertigtes bzw. vorkonfektioniertes Montagemodul.

Zur Endmontage der Massagevorrichtung 1 werden die Massageeinheiten 2 in die hier nicht dargestellten Ausnehmungen 8 des Polsters 3 eingesetzt und mit der Verbindungseinrichtung 5 kontaktiert. Anschließend wird das Polster 3 samt der Massageeinheiten 2, der Verbindungseinrichtung 5 und der Steuervorrichtung 4 mit dem Bezug 11 bedeckt bzw. überspannt. Somit sind in einem im Fahrzeugsitz 10 endmontierten Zustand der Massagevorrichtung 1 die Verbindungseinrichtung 5, die Massageeinheiten 2 und das Steuergerät 4 mitverpolstert.

Fig. 15 zeigt eine Längsschnittansicht eines Fahrzeugsitzes 10 mit einer Massagevorrichtung 1, welche eine Gruppe 9 von Massageeinheiten 2 gemäß Fig. 6 aufweist.

Die Gruppe 9 von Massageeinheiten 2 ist in die Rückenlehne 14 des Fahrzeugsitzes 10 integriert vorgesehen. Die vorliegend dargestellte Gruppe 9 von drei Massageeinheiten 2 ist in einer dafür innerhalb des Polsters 3 der Rückenlehne 14 vorgesehenen Ausnehmung 8 durch Haftreibung selbstbefestigt. Aufgrund eines hohen Reibungskoeffizienten zwischen dem Schaum des Schaumkissens 26 und dem Polster 3 liegt eine Selbstbefestigung vor, wenn die Gruppe 9 von Massageeinheiten 2 mit dem Schaumkissen 26 in die Ausnehmung 8 des Polsters 3, wie dargestellt, eingeführt ist.

Analog zu der Ausführungsform gemäß Fig. 8 mit einer einzelnen Massageeinheit 2 kann auch die Reibungskraft und damit die Selbstbefestigung einer Gruppe 9 verstärkt werden, indem die Größe des Schaumkissens 26 mit einem Übermaß auf die Ausnehmung 8 des Polsters 3 der Rückenlehne 14 abgestimmt wird.

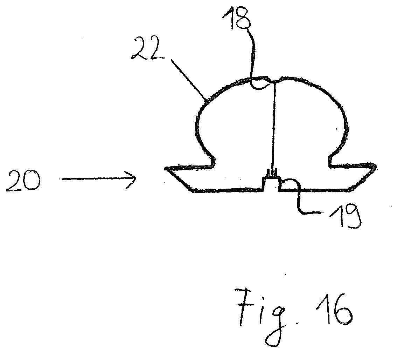

Fig.16 zeigt eine Ausführungsform eines Gehäuses 22 für eine Massageeinheit 2.

Das Gehäuse 22 weist zwei Hälften auf, welche über ein Filmscharnier 18 miteinander verbunden sind. Das Gehäuse 22 ist somit einteilig ausgebildet. Es handelt sich bei den Gehäusen 22 um ein Spritzgussteil. Ferner ist ein mit eingespritzter Stecker 19 vorgesehen. In bei der dargestellten Ausführungsform bildet jede Hälfte des Gehäuses 22 auch eine Hälfte des Steckers 18.

Zur Fertigung einer Massageeinheiten 2 wird ein Vibrationsgenerator 23 derart in das Gehäuse 22 eingelegt, dass er über den Stecker 19 kontaktierbar ist. Anschließend werden die beiden Hälften des Gehäuses 22 zusammengefügt, indem das Filmscharnier 18 umgebogen wird.

Sofern auf die selbstbefestigende Ausbildung 20 eine die beiden Hälften zusammenpressende Kraft wirkt, wird der Stecker 19, wie hier dargestellt, in seine vorgesehene Form zusammengefügt. Die beiden Hälften können zusätzlich miteinander befestigt, insbesondere verklipst, verschraubt oder verschweißt werden.

In der zusammengefügten Konfiguration des Gehäuses 22 kann dann eine Verbindungseinrichtung 5 mit dem Stecker 19 kontaktiert werden.

Obwohl die vorliegende Erfindung anhand bevorzugter Ausführungsbeispiele vorstehend vollständig beschrieben wurde, ist sie darauf nicht beschränkt, sondern auf vielfältige Art und Weise modifizierbar.

Es versteht sich, dass der Sitz 103 nicht nur in einem Fahrzeug angeordnet sein kann. Vielmehr kann der Sitz 103 z.B. auch ein Flugzeugsitz, ein Sitz in einem Zug oder Bus oder dergleichen sein. Wird die vorliegende Erfindung mit einer Vielzahl von Sitzen 103 eingesetzt, kann für jeden der Sitze 103 eine separate Steuervorrichtung 106 vorgesehen sein. Dies ist insbesondere dann vorteilhaft, wenn für jeden Sitz 103 eine eigene Abspielvorrichtung 105 bereitsteht. Dies ist z.B. in Flugzeugen oder Zügen der Fall, in welchen Kopfhörerbuchsen an jedem der Sitze angeordnet sind und ein Benutzer sich einen Audiokanal einstellen kann, welchen er hören möchte. Alternativ kann aber auch eine zentrale Steuervorrichtung 106 vorgesehen sein, die alle Sitze 103 ansteuert.

Ferner können die Gruppen 9 gemäß Figur 6 bzw. 15 in einem Schaumkissen 26 vorkonfektioniert und zudem gemäß Figur 10 vorverkabelt ausgebildet sein.

Es können analog zur Rückenlehne 14 gemäß Fig. 15 auch Gruppen 9 von Massageeinheiten in das Sitzpolster 13 integriert vorgesehen sein.

### Bezugszeichenliste

- 1: Massagevorrichtung
- 2: Massageeinheit
- 3: Polster
- 4: Steuervorrichtung
- 5: Verbindungseinrichtung
- 6: Kabel
- 7: Kabelstrang
- 8: Ausnehmung
- 9: Gruppe
- 10: Fahrzeugsitz
- 11: Bezug
- 12: Basis
- 13: Sitzfläche
- 14: Rückenlehne
- 15: Kopfstütze
- 16: Führungsschiene
- 17: Anschlusspunkt
- 18: Filmscharnier
- 19: Stecker
- 20: selbstbefestigende Ausbildung
- 21: Widerhaken
- 22: Gehäuse
- 23: Vibrationsgenerator
- 24: schräge Stirnfläche
- 25: Kante
- 26: Schaumkissen

- 101: Sitzanordnung
- 102-1 bis 102-n: Massageeinheiten
- 103: Sitz
- 104: Unterhaltungssignal
- 105: Abspielvorrichtung
- 106: Steuervorrichtung
- 107: Ausgangssignal

- S1 - S7: Verfahrensschritte

## Patentansprüche

1. Fahrzeug-Audio-System,
mit einer integrierten Steuervorrichtung (106) zum Steuern von Massageeinheiten (102-1 bis 102-n) in einem Fahrzeugsitz (103), welche dazu ausgebildet ist, ein akustisches Unterhaltungssignal (104) in zumindest ein die Massageeinheiten (102-1 bis 102-n) ansteuerndes Ausgangssignal (107) zu wandeln und die Massageeinheiten (102-1 bis 102-n) basierend auf dem akustischen Unterhaltungssignal (104) anzusteuern.

2. Verfahren zur Ansteuerung von Massageeinheiten (102-1 bis 102-n) in einem Fahrzeugsitz (103) mit einem Fahrzeug-Audio-System gemäß Anspruch 1, mit den folgenden Verfahrensschritten:
Abspielen (S1) eines akustischen Unterhaltungssignals (104), insbesondere eines Musikstücks, mittels einer Abspielvorrichtung (105), insbesondere eines Unterhaltungssystems eines Fahrzeugs; und
Steuern (S2) der in den Fahrzeugsitz (103) integrierten Massageeinheiten (102-1 bis 102-n) basierend auf dem akustischen Unterhaltungssignal (104) durch die Steuervorrichtung (106).

3. Verfahren nach Anspruch 2,
**dadurch gekennzeichnet,**
**dass** das akustische Unterhaltungssignal (104) in vorbestimmte Frequenzbereiche aufgeschlüsselt wird, wobei zumindest ein vorbestimmter Frequenzbereich des akustischen Unterhaltungssignals (104) zu einem Ausgangssignal (107) für zumindest eine Massageeinheit (102-1 bis 102-n) gewandelt wird.

4. Verfahren nach einem der Ansprüche 2 oder 3,
**dadurch gekennzeichnet,**
**dass** verschiedene Frequenzbereiche des akustischen Unterhaltungssignals (104) jeweils zu einem Ausgangssignal (107) gewandelt werden (S3) und zur Ansteuerung verschiedener Massageeinheiten (102-1 bis 102-n) verwendet werden (S2).

5. Verfahren nach Anspruch 4,
**dadurch gekennzeichnet,**
**dass** eine Vielzahl von Massageeinheiten (102-1 bis 102-n) über die Sitzfläche des Sitzes (103) verteilt angeordnet ist und die den verschiedenen Frequenzbereichen des akustischen Unterhaltungssignals (104) zugeordneten Ausgangssignale (107) zur Ansteuerung von an unterschiedlichen Stellen angeordneten Massageeinheiten (102-1 bis 102-n) ausgegeben werden.

6. Verfahren nach einem der Ansprüche 2 bis 5,
**dadurch gekennzeichnet,**
**dass** lediglich ein Teil der in den Sitz (103) integrierten Massageeinheiten (102-1 bis 102-n) mit dem auf dem Unterhaltungssignal (104) basierenden Ausgangssignal (107) angesteuert wird.

7. Verfahren nach einem der Ansprüche 3 bis 6,
**dadurch gekennzeichnet,**
**dass** eine an die Art des Unterhaltungssignals (104), insbesondere an eine Musikart, angepasste Verstärkung und Auswahl der verschiedenen Frequenzbereiche vorgesehen ist und/oder dass die Signale eines Mehrkanalsignals, insbesondere eines Stereo- oder digitalen Mehrkanalsignals, zur Ansteuerung von in einzelnen Bereichen, insbesondere einzelnen Seitenbereichen, des Sitzes (103) angeordneten Massageeinheiten (102-1 bis 102-n) genutzt werden und/oder dass aufeinander folgend stets wechselnde Massageeinheiten (102-1 bis 102-n) angesteuert werden, insbesondere derart, dass die Wechsel für einen Insassen unspürbar sind.

8. Verfahren nach einem der Ansprüche 2 bis 7,
**dadurch gekennzeichnet,**
**dass** das akustische Unterhaltungssignal (104) unabhängig von einer an der Abspielvorrichtung (105) eingestellten Ausgabelautstärke als Eingangsgröße für das Ansteuern verwendet wird.

9. Verfahren nach Anspruch 8,
**dadurch gekennzeichnet,**
**dass** die Stärke der durch die Massageeinheiten (102-1 bis 102-n) erzeugten Massage, insbesondere die Stärke einer Vibration der Massageeinheiten (102-1 bis 102-n), und/oder das verwendete Frequenzband, insbesondere die Lage und Breite der verschiedene Frequenzbereiche des akustischen Unterhaltungssignals (104), die zu einem Ausgangssignal (107) gewandelt werden (S3), und/oder die Verteilung des angesteuerten Teils der Massageeinheiten (102-1 bis 102-n), insbesondere Lage und Anzahl der Massageeinheiten (102-1 bis 102-n), einstellbar ist, vorzugsweise frei einstellbar.

10. Verfahren nach einem der Ansprüche 2 bis 9,
**dadurch gekennzeichnet,**
**dass** das akustische Unterhaltungssignal (104) für die
Ansteuerung der Massageeinheiten (102-1 bis 102-n), insbesondere durch Pulsweitenmodulation, bevorzugt mittels eines einzelnen High-Side-Schalters, in eine Folge von Pulsen gewandelt wird (S4) und/oder dass der Dynamikverlauf des akustischen Unterhaltungssignals (104) für die Ansteuerung komprimiert und/oder expandiert wird (S5).

11. Verfahren nach einem der Ansprüche 2 bis 10,
**dadurch gekennzeichnet,**
**dass** das akustische Unterhaltungssignal (104) mittels einer Audiosignalquelle, insbesondere einer mobilen Audiosignalquelle oder einer fahrzeuginternen Audiosignalquelle, in die Abspielvorrichtung (105) eingespeist wird (S6).

12. Verfahren nach einem der Ansprüche 2 bis 11,
**dadurch gekennzeichnet,**
**dass** die Massageeinheiten (102-1 bis 102-n) zur Vibration in Frequenzbereichen angesteuert werden, in welchen sie nach außen hin für andere als auf dem betroffenen Sitz (103) sitzende Personen, insbesondere für andere Fahrzeuginsassen, lediglich unwahrnehmbare Geräuschemission verursachen, oder
**dass** die Massageeinheiten (102-1 bis 102-n) derart angeordnet und angesteuert werden, dass ihre Vibration von einer elektromotorischen Sitzverstellung gleichzeitig erzeugte Verstellgeräusche überdeckt oder neutralisiert.

13. Verfahren nach einem der Ansprüche 2 bis 12,
**dadurch gekennzeichnet,**
**dass** parallel zur Ansteuerung der Massageeinheiten (102-1 bis 102-n) eine Lautsprecherausgabe des akustischen Unterhaltungssignals (104) erfolgt (S7).

14. Sitzanordnung (106) zur Durchführung eines Verfahrens gemäß einem der vorstehenden Ansprüche 2 bis 13,
mit einem Fahrzeugsitz (103), welcher eine Anzahl von integrierten Massageeinheiten (102-1 bis 102-n) aufweist,
mit einem Fahrzeug-Audio-System gemäß Anspruch 1,
mit einer Abspielvorrichtung (105) zum Abspielen des akustischen Unterhaltungssignals (104), wobei die Abspielvorrichtung (105) mit der Steuervorrichtung (106) zum Steuern der Massageeinheiten (102-1 bis 102-n) gekoppelt ist, so dass sich das die Massageeinheiten (102-1 bis 102-n) ansteuernde Ausgangssignal (107) basierend auf dem Unterhaltungssignal (104) verändert.

15. Sitzanordnung (106) nach Anspruch 14,
**dadurch gekennzeichnet,**
**dass** die Massageeinheiten (102-1 bis 102-n) als in unterschiedlichen Frequenzbereichen betreibbare Unwuchtmotoren, insbesondere Unwucht-Schrittmotoren, ausgebildet sind,
und/oder dass ein Gleichrichterelement, insbesondere eine Diode, in der Motorzuleitung oder in einer Verbindung zwischen der Abspielvorrichtung (105) und der Steuervorrichtung (106) angeordnet ist.

## Claims

1. A vehicle audio system,
comprising an integrated control device (106) for controlling massage units (102-1 to 102-n) in a vehicle seat (103), which is configured to convert an acoustic entertainment signal (104) into at least one output signal (104) driving the massage units (102-1 to 102-n) and to drive the massage units (102-1 to 102-n) based on the acoustic entertainment signal (104).

2. A method of driving massage units (102-1 to 102-n) in a vehicle seat (103) with a vehicle audio system according to claim 1, comprising the following method steps:
playing (S1) back an acoustic entertainment signal (104), in particular a piece of music, by means of a playback device (105), in particular an entertainment system of a vehicle; and
controlling (S2) the massage units (102-1 to 102-n) integrated in the vehicle seat (103) based on the acoustic entertainment signal (104) by means of the control device (106).

3. The method according to claim 2, **characterized in that** the acoustic entertainment signal (104) is broken down into predetermined frequency ranges, wherein at least one predetermined frequency range of the acoustic entertainment signal (104) is converted into an output signal (107) for at least one massage unit (102-1 to 102-n).

4. The method according to any of claims 2 or 3, **characterized in that** different frequency ranges of the acoustic entertainment signal (104) are each converted (S3) into an output signal (107) and used (S2) to control different massage units (102-1 to 102-n).

5. The method according to claim 4, **characterized in that** a plurality of massage units (102-1 to 102-n) are arranged distributed over the seat surface of the seat (103), and the output signals (107) assigned to the different frequency ranges of the acoustic entertainment signal (104) are output for driving massage units (102-1 to 102-n) arranged at different locations.

6. The method according to any of claims 2 to 5, **characterized in that** only some of the massage units (102-1 to 102-n) integrated in the seat (103) are driven by the output signal (107) based on the entertainment signal (104).

7. The method according to any of claims 3 to 6, **characterized in that** an amplification and selection of the various frequency ranges adapted to the type of entertainment signal (104), in particular to a type of music, is provided and/or **in that** the signals of a multichannel signal, in particular a stereo or digital multichannel signal, are used to control massage units (102-1 to 102-n) arranged in individual regions, in particular individual side regions, of the seat (103) and/or **in that** successively constantly changing massage units (102-1 to 102-n) are controlled, in particular in such a way that the changes are imperceptible to an occupant.

8. The method according to any of claims 2 to 7, **characterized in that** the acoustic entertainment signal (104) is used as an input variable for driving independently of an output volume set on the playback device (105).

9. The method according to claim 8, **characterized in that** the strength of the massage produced by the massage units (102-1 to 102-n), in particular the strength of a vibration of the massage units (102-1 to 102-n), and/or the frequency band used, in particular the position and width of the different frequency ranges of the acoustic entertainment signal (104), which are converted (S3) into an output signal (107), and/or the distribution of the controlled part of the massage units (102-1 to 102-n), in particular the position and number of the massage units (102-1 to 102-n), is adjustable, preferably freely adjustable.

10. The method according to any of claims 2 to 9, **characterized in that**, for the actuation of the massage units (102-1 to 102-n), the acoustic entertainment signal (104) is converted into a sequence of pulses, in particular by pulse width modulation, preferably by means of a single high-side switch (S4), and/or **in that** the dynamic curve of the acoustic entertainment signal (104) for the actuation is compressed and/or expanded (S5).

11. The method according to any of claims 2 to 10, **characterized in that** the acoustic entertainment signal (104) is fed into the playback device (105) by means of an audio signal source, in particular a mobile audio signal source or an in-vehicle audio signal source (S6).

12. The method according to any of claims 2 to 11, **characterized in that** the massage units (102-1 to 102-n) are driven for vibration in frequency ranges in which they cause only imperceptible noise emission to the outside for persons other than those sitting on the seat (103) concerned, in particular for other vehicle occupants, or **in that** the massage units (102-1 to 102-n) are arranged and driven in such a way that their vibration masks or neutralizes adjustment noises generated simultaneously by an electromotive seat adjustment.

13. The method according to any of claims 2 to 12, **characterized in that** a loudspeaker output of the acoustic entertainment signal (104) takes place (S7) in parallel with the activation of the massage units (102-1 to 102-n).

14. A seat arrangement (106) for carrying out a method according to any of the preceding claims 2 to 13,
comprising a vehicle seat (103) having a number of integrated massage units (102-1 to 102-n),
comprising a vehicle audio system according to claim 1 having a playback device (105) for playing back the acoustic entertainment signal (104), wherein the playback device (105) is coupled to the control device (106) for controlling the massage units (102-1 to 102-n) so that the output signal (107) driving the massage units (102-1 to 102-n) varies based on the entertainment signal (104).

15. The seat arrangement (106) according to claim 14, **characterized in that** the massage units (102-1 to 102-n) are designed as unbalanced motors, in particular unbalanced stepper motors, which can be operated in different frequency ranges,
and/or **in that** a rectifier element, in particular a diode, is arranged in the motor supply line or in a connection between the playback device (105) and the control device (106).

## Revendications

1. Un système audio du véhicule,
avec un dispositif de commande intégré (106) pour commander des unités de massage (102-1 à 102-n) dans un siège de véhicule (103), qui est conçu pour convertir un signal de divertissement acoustique (104) en au moins un signal de sortie (107) commandant les unités de massage (102-1 à 102-n) et pour commander les unités de massage (102-1 à 102-n) sur la base du signal de divertissement acoustique (104).

2. Un procédé de commande d'unités de massage (102-1 à 102-n) dans un siège de véhicule (103) ayant un système audio de véhicule selon la revendication 1, comprenant les étapes de procédé suivantes :
lire (S1) un signal de divertissement acoustique (104), en particulier un morceau de musique, au moyen d'un dispositif de lecture (105), en particulier un système de divertissement d'un véhicule ; et
commander (S2) des unités de massage (102-1 à 102-n) intégrées dans le siège de véhicule (103) sur la base du signal de divertissement acoustique (104) par le dispositif de commande (106).

3. Le procédé selon la revendication 2, **caractérisé en ce que** le signal de divertissement acoustique (104) est décomposé en plages de fréquence prédéterminées, dans lequel au moins une plage de fréquence prédéterminée du signal de divertissement acoustique (104) est convertie en un signal de sortie (107) pour au moins une unité de massage (102-1 à 102-n).

4. Le procédé selon l'une quelconque des revendications 2 ou 3, **caractérisé en ce que** différentes plages de fréquences du signal de divertissement acoustique (104) sont chacune converties (S3) en un signal de sortie (107) et utilisées (S2) pour commander différentes unités de massage (102-1 à 102-n).

5. Le procédé selon la revendication 4, **caractérisé en ce qu'**une pluralité d'unités de massage (102-1 à 102-n) sont disposées réparties sur la surface d'assise du siège (103) et les signaux de sortie (107) associés aux différentes plages de fréquence du signal de divertissement acoustique (104) sont émis pour commander des unités de massage (102-1 à 102-n) disposées à différents endroits.

6. Le procédé selon l'une quelconque des revendications 2 à 5, **caractérisé en ce que** seule une partie des unités de massage (102-1 à 102-n) intégrées dans le siège (103) est commandée par le signal de sortie (107) sur la base du signal de divertissement (104).

7. Le procédé selon l'une quelconque des revendications 3 à 6, **caractérisé en ce qu'**une amplification et une sélection des différentes plages de fréquences adaptées au type de signal de divertissement (104), notamment à un type de musique, sont prévues et/ou **en ce que** les signaux d'un signal multicanal, notamment un signal multicanal stéréo ou numérique, sont utilisées pour commander des unités de massage (102-1 à 102-n) disposées dans des régions individuelles, en particulier des régions latérales individuelles, du siège (103) et/ou **en ce que** des unités de massage (102-1 à 102-n) changeant constamment de manière successive sont commandées, en particulier de telle manière que les changements sont imperceptibles pour un occupant.

8. Le procédé selon l'une quelconque des revendications 2 à 7, **caractérisé en ce que** le signal de divertissement acoustique (104) est utilisé comme grandeur d'entrée pour le déclenchement indépendamment d'un volume de sortie réglé sur le dispositif de lecture (105).

9. Le procédé selon la revendication 8, **caractérisé en ce que** l'intensité du massage produit par les unités de massage (102-1 à 102-n), en particulier l'intensité d'une vibration des unités de massage (102-1 à 102-n), et/ou la bande de fréquence utilisée, en particulier la position et la largeur des différentes plages de fréquence du signal de divertissement acoustique (104), qui sont convertis (S3) en un signal de sortie (107), et/ou la répartition de la partie commandée des unités de massage (102-1 à 102-n), en particulier la position et le nombre des unités de massage (102-1 à 102-n), est réglable, de préférence librement réglable.

10. Le procédé selon l'une quelconque des revendications 2 à 9, **caractérisé en ce que** le signal de divertissement acoustique (104) pour la commande des unités de massage (102-1 à 102-n) est converti en une séquence d'impulsions, en particulier par modulation de largeur d'impulsion, de préférence au moyen d'un seul commutateur côté haut (S4), et/ou **en ce que** la réponse dynamique du signal de divertissement acoustique (104) pour la commande est comprimée et/ou étendue (S5).

11. Le procédé selon l'une quelconque des revendications 2 à 10, **caractérisé en ce que** le signal de divertissement acoustique (104) est introduit (S6) dans le dispositif de lecture (105) au moyen d'une source de signal audio, notamment une source de signal audio mobile ou une source de signal audio embarquée.

12. Le procédé selon l'une quelconque des revendications 2 à 11, **caractérisé en ce que** les unités de massage (102-1 à 102-n) sont entraînées en vibration dans des plages de fréquences dans lesquelles elles ne provoquent qu'une émission de bruit imperceptible vers l'extérieur pour des personnes autres que celles assises sur le siège (103) concerné, en particulier pour d'autres occupants du véhicule, ou **en ce que** les unités de massage (102-1 à 102-n) sont disposées et entraînées de telle sorte que leur vibration masque ou neutralise les bruits de réglage générés simultanément par un réglage électromoteur du siège.

13. Le procédé selon l'une quelconque des revendications 2 à 12, **caractérisé en ce qu'**une émission par haut-parleur du signal de divertissement acoustique (104) est effectuée (S7) en parallèle avec la commande des unités de massage (102-1 à 102-n).

14. Une agencement de siège (106) pour la mise en oeuvre d'un procédé selon l'une quelconque des revendications précédentes 2 à 13,
comprenant un siège de véhicule (103) ayant un certain nombre d'unités de massage intégrées (102-1 à 102-n),
avec un système audio du véhicule selon la revendication 1, comprenant un dispositif de lecture (105) pour lire le signal de divertissement acoustique (104), dans lequel le dispositif de lecture (105) est couplé au dispositif de commande (106) pour commander les unités de massage (102-1 à 102-n) de sorte que le signal de sortie (107) commandant les unités de massage (102-1 à 102-n) varie en fonction du signal de divertissement (104).

15. L'agencement de siège (106) selon la revendication 14, **caractérisé en ce que** les unités de massage (102-1 à 102-n) sont conçues comme des moteurs à balourd, en particulier des moteurs pas à pas à balourd, qui peuvent fonctionner dans différentes plages de fréquence,
et/ou **en ce qu'**un élément redresseur, en particulier une diode, est disposé dans la ligne d'alimentation du moteur ou dans une liaison entre le dispositif de lecture (105) et le dispositif de commande (106).
